# EUROPEAN PATENT SPECIFICATION

(11) **EP 4 499 056 B1**
(45) Date of publication and mention of the grant of the patent: **09.12.2026**
(21) Application number: 23728596.0
(22) Date of filing: 30.03.2023
(51) Int. Cl.: A61K 31/137, A61K 31/397, A61K 31/4245, A61K 45/06, A61P 25/28

(54) **A SPHINGOSINE-1-PHOSPHATE RECEPTOR (S1PR) MODULATOR OZANIMOD FOR USE IN TREATING A PATIENT SUFFERING FROM ALZHEIMER'S DEMENTIA**
SPHINGOSIN-1-PHOSPHAT-REZEPTOR(S1PR)-MODULATOR OZANIMOD ZUR VERWENDUNG BEI DER BEHANDLUNG EINES PATIENTEN MIT ALZHEIMER-DEMENZ
MODULATEUR DU RÉCEPTEUR DE SPHINGOSINE-1-PHOSPHATE (S1PR) OZANIMOD DESTINÉ À ÊTRE UTILISÉ DANS LE TRAITEMENT D'UN PATIENT SOUFFRANT DE DÉMENCE D'ALZHEIMER

(30) Priority: 31.03.2022 EP 22166162
(43) Date of publication of application: 05.02.2025
(73) Proprietor: Otto-von-Guericke-Universität Magdeburg, 39106 Magdeburg (DE)
(72) Inventor: LESSMANN, Volkmar, 39108 Magdeburg (DE); GOTTMANN, Kurt, 79341 Kenzingen (DE); ENDRES, Thomas, 39116 Magdeburg (DE); KARTALOU, Georgia-Ioanna, 11528 Athens (GR)
(74) Representative: Dr. Karen Braeuning
(86) International application number: PCT/EP2023/058360
(87) International publication number: WO 2023/187091

(56) References cited:
- WO-A1-2016/044103
- KARTALOU GEORGIA-IOANNA ET AL: "Anti-Inflammatory Treatment with FTY720 Starting after Onset of Symptoms Reverses Synaptic Deficits in an AD Mouse Model", INTERNATIONAL JOURNAL OF MOLECULAR SCIENCES, vol. 21, no. 23, 25 November 2020 (2020-11-25), pages 8957, XP055956523, DOI: 10.3390/ijms21238957
- FAGAN STEVEN G ET AL: "Fingolimod Rescues Memory and Improves Pathological Hallmarks in the 3xTg-AD Model of Alzheimer's Disease", MOLECULAR NEUROBIOLOGY, SPRINGER US, NEW YORK, vol. 59, no. 3, 15 January 2022 (2022-01-15), pages 1882 - 1895, XP037703483, ISSN: 0893-7648, [retrieved on 20220115], DOI: 10.1007/S12035-021-02613-5
- CARRERAS ISABEL ET AL: "Dual dose-dependent effects of fingolimod in a mouse model of Alzheimer's disease", vol. 9, no. 1, 29 July 2019 (2019-07-29), XP055956311, Retrieved from the Internet <URL:http://www.nature.com/articles/s41598-019-47287-1> DOI: 10.1038/s41598-019-47287-1
- ANGELOPOULOU EFTHALIA ET AL: "Beneficial Effects of Fingolimod in Alzheimer's Disease: Molecular Mechanisms and Therapeutic Potential", NEUROMOLECULAR MEDICINE, HUMANA PRESS, US, vol. 21, no. 3, 16 July 2019 (2019-07-16), pages 227 - 238, XP036858747, ISSN: 1535-1084, [retrieved on 20190716], DOI: 10.1007/S12017-019-08558-2
- PATNAIK ABHISARIKA ET AL: "Fingolimod Modulates Dendritic Architecture in a BDNF-Dependent Manner", INTERNATIONAL JOURNAL OF MOLECULAR SCIENCES, vol. 21, no. 9, 27 April 2020 (2020-04-27), pages 3079, XP055956557, DOI: 10.3390/ijms21093079
- ASLE-ROUSTA MASOUMEH ET AL: "ACTIVATION OF SPHINGOSINE 1-PHOSPHATE RECEPTOR-1 BY SEW2871 IMPROVES COGNITIVE FUNCTION IN ALZHEIMER?S DISEASE MODEL RATS", EXCLI JOURNAL, 3 June 2013 (2013-06-03), pages 449 - 461, XP055956320, Retrieved from the Internet <URL:https://pubmed.ncbi.nlm.nih.gov/26417237/> [retrieved on 20220830]
- FESSEL JEFFREY: "Reversing Alzheimer's disease dementia with clemastine, fingolimod, or rolipram, plus anti-amyloid therapy", vol. 8, no. 1, 1 January 2022 (2022-01-01), XP055956551, ISSN: 2352-8737, Retrieved from the Internet <URL:https://onlinelibrary.wiley.com/doi/full-xml/10.1002/trc2.12242> DOI: 10.1002/trc2.12242

## Description

The present invention relates to a sphingosine-1-phosphate receptor (S1PR) modulator for use in treating a patient suffering from Alzheimer's dementia in a late stage of Alzheimer's disease, wherein said S1PR modulator is ozanimod (RPC1063).

### BACKGROUND

Dementia is one of the greatest global challenges for health and social care in the 21st century. It occurs mainly in people older than 65 years. Globally, about 50 million people are currently living with dementia, and this number is projected to triple by 2050. The 2015 global cost of dementia was >800 billion € per year, and this figure will continue to increase as the number of people with dementia rises.

Alzheimer's disease (AD) is the most common (~60%) form of dementia characterized by a progressive decline of cognitive functions. Pathologically, AD is characterized by amyloid plaques and neurofibrillary tangles in the brain, with associated loss of synapses and neurons, resulting in cognitive deficits and eventually dementia. While lifestyle factors such as physical exercise or a healthy diet might be able to delay the disease onset, >30 years of dementia research have only very recently led to a single drug (aducanumab, FDA approved 2021) for causal treatment of dementia in AD patients.

Increasing evidence suggests that AD pathogenesis is not restricted to neurons, but includes strong interactions with immunological mechanisms in the brain. Misfolded and aggregated proteins bind to pattern recognition receptors on microglia and astroglia, and trigger an innate immune response characterized by the release of inflammatory mediators, which contribute to disease progression and severity. This inflammatory response is also present in the ~40% dementia patients that cannot be assigned unequivocally as AD patients, but which show similar synaptic dysfunctions, and would therefore also benefit from treatments which tackle neuroinflammation. Accordingly, therapeutic approaches focusing on reduction in neuroinflammatory signaling are among the most promising interventions to ameliorate AD-related deficits, and deficits observed in other types of dementia (e.g., vascular dementia, mixed AD and vascular dementia, frontotemporal dementia, dementia with Lewy bodies). Therefore, repurposing FDA-approved anti-inflammatory drugs for therapy of AD and other types of dementia could provide an important benefit to rapidly develop an effective AD/dementia medication.

Fingolimod (FTY720, Gilenya^{®}) is an approved (FDA and EMA) drug for the treatment of relapsing multiple sclerosis (RMS) since 2011. In target tissue, it can be converted to fingolimod-phosphate, which is a potent modulator of sphingosine-1-phosphate receptors (S1PR1-5). The sphingosine-1-phosphate receptors are a family of seven helix transmembrane G protein-coupled receptors that recognize and bind extracellular sphingosine-1-phosphate to affect cellular processes. They are divided into five subtypes: S1PR1, S1PR2, S1PR3, S1PR4, and S1PR5. In the immune system, fingolimod-induced S1PR receptor modulation promotes the retention of T-cells in lymph nodes, thereby reducing the invasion of the central nervous system by auto-reactive lymphocytes, where they attack the protective sheath (myelin) that covers nerve fibers and cause permanent damage or deterioration of the nerves.
Kartalou *et al.* disclose that the S1PR modulator fingolimod reverses synaptic deficits in five months old AD mice. The treatment started after the onset of symptoms (at five months of age) and was set forth for one to two months.

At present, there is no treatment for Alzheimer's dementia known. In particular, there is no treatment for Alzheimer's dementia known that can stop AD progression if the disease is not in its onset but already in a late stage.

The present invention is based on the surprising finding that treatment with a modulator of sphingosine-1-phosphate receptor, namely ozanimod (RPC1063), may stop progression of synaptic deficits and memory dysfunction in a late state of AD. Even more surprising is the finding, that treatment with the S1PR modulator ozanimod is even effective to revert AD. The S1PR modulator ozanimod can also be used in combination therapy.

### SUMMARY OF THE INVENTION

In a first aspect, the present invention relates to a sphingosine-1-phosphate receptor (S1PR) modulator for use in treating a patient suffering from Alzheimer's dementia in a late stage of Alzheimer's disease, wherein said S1PR modulator is ozanimod (RPC1063).

In a second aspect, the invention relates to a combination for use in treating a patient suffering from Alzheimer's dementia in a late stage of Alzheimer's disease, wherein the combination comprises
(i) an S1PR modulator, and
(ii) another therapeutic substance,
wherein said S1PR modulator is ozanimod (RPC1063).
The other therapeutic substance may be selected from the group consisting of aducanumab, lecanemab, gantenerumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

In a third aspect, the present invention relates to a pharmaceutical composition comprising the sphingosine-1-phosphate receptor (S1PR) modulator as defined in the first aspect or the combination as defined in the second aspect for use in treating a patient suffering from Alzheimer's dementia in a late stage of Alzheimer's disease.

### DETAILED DESCRIPTION

### Definitions

In the following the invention is described in more detail with reference to the Figures. The described specific embodiments of the invention, examples, or results are, however, intended for illustration only and should not be construed to limit the scope of the invention as indicated by the appended claims in any way.

It is to be understood that this invention is not limited to the particular methodology, protocols, and reagents described herein as these may vary. It is also to be understood that the terminology used herein is to describe particular embodiments only, and is not intended to limit the scope of the present invention, which will be limited only by the appended claims. Unless defined otherwise, all technical and scientific terms used herein have the same meanings as commonly understood by one of ordinary skill in the art.

The term "comprising" or variations thereof such as "comprise(s)" according to the present invention (especially in the context of the claims) is to be construed as an open-ended term or non-exclusive inclusion, respectively (i.e., meaning "including, but not limited to,") unless otherwise noted.

The terms "a", "an", and "the" as used herein in the context of describing the invention (especially in the context of the claims) should be read and understood to include at least one element or component, respectively, and are to be construed to cover both the singular and the plural, unless otherwise indicated herein or clearly contradicted by context.

In addition, unless expressly stated to the contrary, the term "or" refers to an inclusive "or" and not to an exclusive "or" (i.e., meaning "and/or").

All numeric values are herein assumed to be modified by the term "about", whether or not explicitly indicated. Recitation of ranges of values herein is merely intended to serve as a shorthand method of referring individually to each separate value falling within the range, unless otherwise indicated herein, and each separate value is incorporated into the specification as if it were individually recited herein.

The use of terms "for example", "e.g.", "such as", or variations thereof is intended merely to better illuminate the invention and does not pose a limitation on the scope of the invention unless otherwise claimed. These terms should be interpreted to mean "but not limited to" or "without limitation".

The term "dementia", as used herein, is a general term for a decline in mental ability severe enough to interfere with daily life. Dementia describes a group of symptoms associated with a decline in memory, reasoning or other thinking skills. Many different types of dementia exist, and many conditions cause it. Mixed dementia is a condition in which brain changes of more than one type of dementia occur simultaneously. Alzheimer's disease is the most common cause of dementia, accounting for 60-80% (i.e. AD, or mixed AD + vascular dementia) of dementia cases. Alzheimer's is a specific disease. Dementia is not.

The term "Alzheimer's disease (AD)", as used herein, refers to a neurodegenerative disease. It is the most common form of dementia. Alzheimer's disease is characterized by loss of neurons and synapses in the cerebral cortex and certain subcortical regions and leads to a gross degeneration in these regions. In Alzheimer's disease, protein misfolding and aggregation (formation of so-called "plaques") in the brain is caused by accumulation of abnormally folded A-beta and tau proteins in the affected tissues. The most common early symptom is difficulty in remembering recent events (short-term memory loss). As the disease advances, symptoms can include problems with language, disorientation, mood swings, loss of motivation, not managing self-care and behavioral issues. As a person's condition declines, they often withdraw from family and society. Gradually, bodily functions are lost, ultimately leading to death.

The term "Alzheimer's dementia", as used herein, refers to a specific form of Alzheimer's disease.

Alzheimer's disease (AD), the major cause of dementia among the elderly world-wide, manifests in familial and sporadic forms, and the latter variety accounts for the majority of the patients affected by this disease.

The term "sporadic Alzheimer's dementia", as used herein, refers to a multi-factorial disorder in which both genetic predisposition and environmental factors contribute to the genesis of the disease. The majority (more than 95 %) of the AD dementia cases belong to sporadic variety of the disease. Sporadic Alzheimer's disease can affect adults at any age, but usually occurs after age 65.

The term "familial Alzheimer's dementia", as used herein, refers to a disease which arises from the mutations of any of the three genes e.g. amyloid precursor protein (*APP*), presenilin 1 (*PS 1*) and presenilin 2 (*PS 2*) and usually appears in somewhat younger age-group than the sporadic form and follows a more aggressive downhill course. The minority (less than 5%) of the AD dementia cases belong to familial Alzheimer's dementia.

The term "Sphingosine-1-phosphate (SIP)", as used herein, refers to a signaling molecule involved in, among other things, lymphocyte migration, brain and heart development, vascular permeability, and regulation of vascular and bronchial tone. In this regard, S1P acts through a series of membrane-bound S1P receptors, in particular S1P receptor subtypes (S1PR1, 2, 3, 4, 5) that exhibit variable organ- and cell-specific expression patterns and thus have distinct functions.

The term "Sphingosine-1-phosphate receptor (S1PR)", as used herein, refers to a receptor bound by S1P.

In biochemistry and pharmacology, a "receptor modulator" is a general term for a substance, endogenous or exogenous, that binds to and regulates the activity of certain cell receptors, i.e. protein structures that receive and transduce signals and cause some form of cellular response. Receptor modulators can act on different parts of receptors and regulate activity in a positive, negative, or neutral direction with varying degrees of efficacy. Categories of these receptor modulators include receptor agonists and receptor antagonists. Generally, an agonist is a modulator (e.g. drug) that binds to a receptor and produces a similar response to the intended (natural) binding ligand. An antagonist is a modulator (e.g. drug) that binds to the receptor and stops the receptor from producing a cellular response.

The term "Sphingosine-1-phosphate receptor (S1PR) modulator", as used herein, refers to molecules that bind to S1P receptors, e.g. on lymphocytes, and lead to internalization of the receptor. It is thought that there is a subsequent loss of sensitivity to the S1P gradient, which causes the lymphocytes to remain in the lymph nodes. Their circulating numbers decrease and inflammatory cell migration to the central nervous system decreases. In addition, direct effects of S1PR modulators on the blood-brain barrier, microglia, astrocytes, and oligodendrocytes may be relevant.

The S1PR modulator used in the present invention is ozanimod (RPC1063). The following S1PR modulators are also known: fingolimod (FTY720), siponimod (BAF312), ceralifimod (ONO-4641), GSK2018682, ponesimod (ACT128800), and amiselimod (MT-1303).

The term "patient", as used herein, refers to any individual, that/who may receive the S1PR modulator or the combination comprising an S1PR modulator and another therapeutic substance, as described herein. In particular, the term "subject", as used herein, refers to any individual that/who may benefit from the treatment with the the S1PR modulator or the combination comprising an S1PR modulator and another therapeutic substance, as described herein. Specifically, the term "patient" refers to a subject that is or will be receiving, or has received, medical treatment for a disease or condition, i.e. Alzheimer's dementia in the context of the present invention. Preferably, the Alzheimer's dementia is sporadic Alzheimer's dementia or familial Alzheimer's dementia.

The patient may be a vertebrate, e.g. a human being, dog, cat, sheep, goat, cow, horse, camel or pig. It is particularly preferred that the "patient" is a human being.

The term "treatment", in particular "therapeutic treatment", as used herein, refers to any therapy which improves the health status and/or prolongs (increases) the lifespan of a subject suffering from a disease. Said therapy may eliminate the disease in a subject, arrest or slow the development of the disease in a subject, inhibit the development of the disease in a subject, decrease the severity of symptoms in a subject suffering the disease, and/or decrease the recurrence in a subject who currently has or who previously has had a disease.

As used herein, the expressions "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity. In the context of the present invention, the S1PR modulator or a composition comprising the S1PR modulator, as described herein, can be prepared for administration.

In the present invention, S1PR modulators, or compositions comprising them, are generally administered in a "therapeutically (pharmaceutically) effective" amount. The term "therapeutically (pharmaceutically) effective" amount, as used herein, generally refers to the amount of a drug that achieves the desired reaction or the desired effect alone or together with further doses. In the case of the treatment of a particular disease, the desired reaction preferably relates to an inhibition of the course of the disease. This comprises slowing down the progress of the disease and, in particular, interrupting or reversing the progress of the disease. The desired reaction in treatment of a disease may also be a delay of the onset or prevention of the onset of the disease. A "therapeutically (pharmaceutically) effective" amount of a drug will generally depend on the condition of the patient to be treated, the severeness of the disease, the individual parameters of the patient, including age, physiological condition, size, and weight, the duration of treatment, the type of an accompanying therapy (if present), the specific route of administration, and similar factors. In case reaction of the patient is insufficient with an initial dose, higher doses (or effectively higher doses achieved by a different, more localized route of administration) may be used.

The pharmaceutical composition as described herein preferably further comprises one or more pharmaceutically acceptable excipients, diluents, and/or carriers.

The term "excipient", as used herein, is intended to indicate all substances in a pharmaceutical composition which are not active ingredients such as binders, lubricants, thickeners, surface active agents, preservatives, emulsifiers, buffers, flavoring agents, or colorants.

The term "diluent", as used herein, relates to a diluting and/or thinning agent. Moreover, the term "diluent" includes a solution, suspension (e.g. liquid or solid suspension) and/or media.

The term "carrier", as used herein, relates to one or more compatible solid or liquid fillers, which are suitable for an administration, e.g. to a human. The term "carrier" relates to a natural or synthetic organic or inorganic component, which is combined with an active component in order to facilitate the application of the active component, or the permeation of the active component to the intended site of action. Preferably, carrier components are sterile liquids such as water or oils, including those which are derived from mineral oil, animals, or plants, such as peanut oil, soy bean oil, sesame oil, sunflower oil, etc. Salt solutions and aqueous dextrose and glycerin solutions may also be used as aqueous carrier compounds. A carrier compound that improves or facilitates permeation of the skin in topical applications is dimethyl sulfoxide (DMSO). Another preferred carrier consists of layered double hydroxide (LDH) nanoparticles. For example, such an LDH nanoparticles can be of the form [Mg3Al(OH)8](CH3CHOHCOO), that can be obtained, for example, by a reaction of Mg(lactate)₂ 3H2O and Al(lactate)₃ in NaOH-controlled pH at 45-65 °C and protected from CO₂ and carbonic acids. A circularized RNA may intercalate into LDH nanoparticles and can be administered as LDH nanoparticle or be co-administered with VLPs.

Pharmaceutical carriers, diluents, and/or excipients can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, lactose, sucrose, trehalose, corn sweeteners, natural and synthetic gums, such as acacia, tragacanth or sodium alginate, carboxymethyl cellulose, and polyethylene glycol. Examples of suitable lubricants include sodium oleate, sodium stearate, magnesium stearate, sodium benzoate, sodium acetate, sodium chloride, and the like. Preservatives, stabilizers, dyes, and even flavoring agents may be provided in the pharmaceutical composition. Examples of preservatives include sodium benzoate, sorbic acid, and esters of p-hydroxybenzoic acid. Antioxidants and suspending agents may be also used.

No language in this specification should be construed as indicating any non claimed element as essential to the practice of the invention.

### Embodiments

The present invention is based on the surprising finding that treatment with the modulator of sphingosine-1-phosphate receptor, namely ozanimod (RPC 1063) may stop progression of synaptic deficits and memory dysfunction in a late state of AD. Even more surprising is the finding, that treatment with said S1PR modulator is even effective to revert AD. The S1PR modulator can also be used in combination therapy.

Thus, in a first aspect, the invention relates to a sphingosine-1-phosphate receptor (S1PR) modulator for use in treating a patient suffering from Alzheimer's dementia in a late stage of Alzheimer's disease, wherein the S1PR modulator is ozanimod (RPC1063). Further known and investigated S1PR modulators are fingolimod (FTY720), siponimod (BAF312), ceralifimod (ONO-4641), GSK2018682, ponesimod (ACT128800), and amiselimod (MT-1303).

Fingolimod (FTY720, tradename Gilenya^{®}) has the formula:

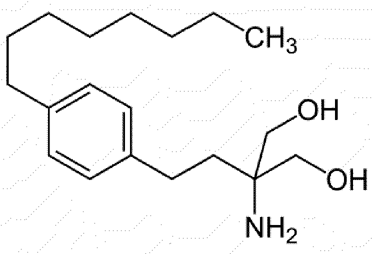

Siponimod (BAF312) has the formula:

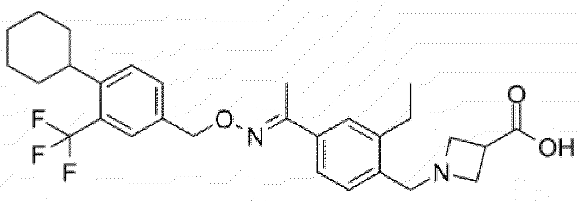

Ozanimod (RPC1063, tradename Zeposia^{®}) has the formula:

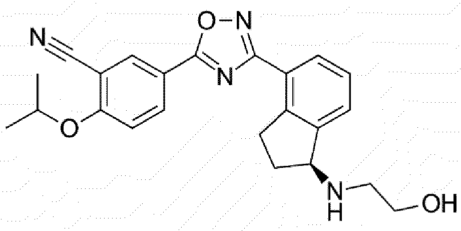

Ceralifimod (ONO-4641) has the formula:

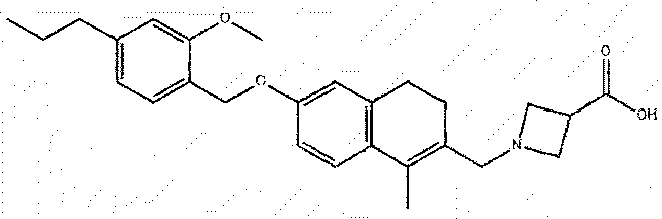

GSK2018682 has the formula:

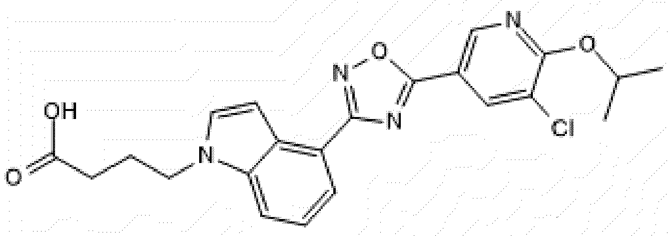

Ponesimod (ACT128800) has the formula:

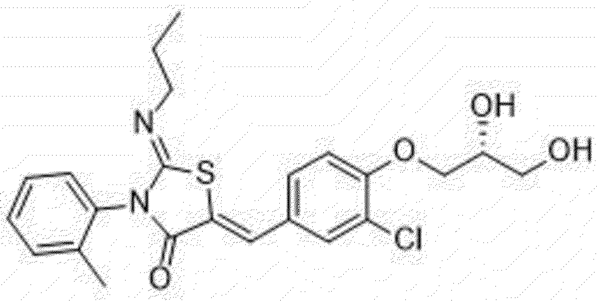

Amiselimod (MT-1303) has the formula:

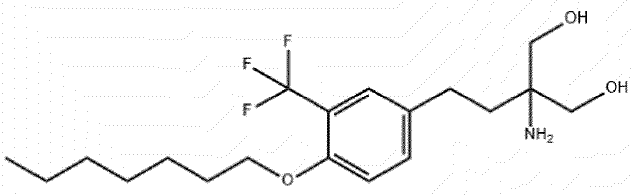

The above S1PR modulators are so far used as medicaments to treat multiple sclerosis or inflammatory bowel disease.

The skilled person appreciates that certain of the above S1PR modulators may have different optical isomers as a result of one or more asymmetric or chiral carbon atoms. Further, some S1PR modulators may display polymorphism. It is also clear that pharmaceutically acceptable salts, esters, solvates, or hydrates of the above S1PR modulators as well as prodrugs that may convert *in vivo* into the above S1PR modulators may be used in the invention.

Examples for pharmacologically acceptable salts of the above S1PR modulators are salts of sufficiently basic S1PR modulators with physiologically acceptable mineral acids like hydrochloric, sulfuric or phosphoric acid; or salts of organic acids like acetic, lactic, citric, succinic, fumaric, maleic or salicylic acid. Further, sufficiently acid S1PR modulators may form alkali or earth alkaline metal salts, for example, sodium, potassium, lithium, calcium or magnesium salts; ammonium salts; or salts of an organic base, for example, methylamine, dimethylamine, trimethylamine, triethylamine, ethylenediamine, ethanolamine, choline hydroxide, piperidine, morpholine, tris-(2-hydroxyethyl)amine, lysine or arginine salts.

Pharmacologically acceptable esters of the above S1PR modulators are esters that can be hydrolyzed by esterase enzyms in the human body into a S1PR modulator with the above formula.

Pharmacologically acceptable solvates of the above S1PR modulators are complex compounds (adducts) formed by the interaction of the molecules of a solvent with molecules of the above S1PR modulators. Hydrates are solvates where the solvent is water.

According to the invention, the sphingosine-1-phosphate receptor (S1PR) modulator ozanimod may be used in treating a patient suffering from Alzheimer's dementia, wherein said patient is about 40 years old or older.

Alzheimer's dementia to be treated may be sporadic Alzheimer's dementia or familial Alzheimer's dementia. Familial Alzheimer's disease is a rare special form (1-2%) of classic Alzheimer's disease with an earlier onset of the disease and may start at an age of about 40 years, while sporadic Alzheimer's disease starts usually at an age of 60 or older.

Thus, according to the invention, ozanimod may be used in treating a patient suffering from sporadic Alzheimer's dementia, wherein the patient may be about 60 years or older, or, in case of a patient suffering from familial Alzheimer's dementia, the patient may be about 40 years or older.

Accordingly, in a more preferred embodiment, the sphingosine-1-phosphate receptor (S1PR) modulator for use in treating a patient suffering from Alzheimer's dementia is ozanimod (RPC1063), wherein the Alzheimer's dementia is sporadic Alzheimer's dementia and wherein the patient is about 60 years or older.

In another more preferred embodiment, the sphingosine-1-phosphate receptor (S1PR) modulator for use in treating a patient suffering from Alzheimer's dementia is ozanimod (RPC1063), wherein the Alzheimer's dementia is familial Alzheimer's dementia and wherein the patient is about 40 years or older.

The skilled person appreciates that the above ages of the Alzheimer patients are only very approximate, as the boundaries between the outbreak of the diseases can overlap.

In one embodiment of the invention, the patient to be administered the sphingosine-1-phosphate receptor (S1PR) modulator ozanimod is a patient who has received or is receiving behavioral therapy, physical exercise training, reality orientation training, physiotherapy, occupational therapy, or cognitive training (memory training), or a combination thereof.

According to the invention, the S1PR modulator ozanimod may be used to reverse Alzheimer's dementia. This is a very surprising finding of the invention.

In the invention, the administration route of an S1PR modulator is not subject to any particular restrictions and may be enteral or parenteral. Examples are intraperitoneal or oral administration.

The skilled person appreciates that S1PR modulators may be administered as such or in the form of a pharmaceutical composition or formulation comprising an S1PR modulator as the active agent and, optionally, pharmaceutically acceptable excipients commonly used in galenics (e.g., carriers, fillers, diluents, absorbents, binders, adjuvants, etc).

For oral administration, the S1PR modulators may be formulated, for example, as tablets (including effervescent tablets, sustained-release tablets, controlled-release tablets, or entericcoated tablets), dragees, pills, granules, powders, gels, semisolids, capsules (including soft and hard capsules, sustained-release capsules, controlled-release capsules or enteric coated capsules). The above oral administration forms may be prepared following standard galenic procedures, for example, using a pharmaceutically acceptable carrier well known in the art. The pharmaceutically acceptable carriers may include fillers, absorbents, wetting agents, binders, disintegrants, lubricants, and the like. The fillers may include starch, lactose, mannitol, microcrystalline cellulose, and the like. The absorbents may include calcium sulfate, calcium hydrogen phosphate, calcium carbonate, and the like. The wetting agents may include water, ethanol, and the like. The binders may include hydroxypropyl methylcellulose, polyvinylpyrrolidone (povidon, INN), microcrystalline cellulose, and the like. The disintegrants may include croscarmellose sodium, crospovidone, surfactants, low-substituted hydroxypropyl cellulose, and the like. The lubricants may include magnesium stearate, talcum powder, polyethylene glycol, sodium dodecyl sulfate, colloidal silicon dioxide, talcum powder, and the like. Also, liquids (e.g. aqueous or oily solutions), emulsions, suspensions, or syrups are suitable for oral administration.

For intraperitoneal administration, the S1PR modulators may be formulated, for example, as an injectable solution, emulsion, or suspension. A suitable solvent is, e.g., 3% dimethyl sulfoxide (DMSO).

The pharmaceutical compositions or formulations comprising S1PR modulators may also contain additives used in galenics such as preservation agents, stabilizers, e.g. UV stabilizers, emulsifiers, sweeteners, flavours, coloring agents, salts to change the osmotic pressure, buffers, antioxidants, and the like.

According to the invention, the S1PR modulator ozanimod for use in treating a patient suffering from Alzheimer's disease may be administered in an amount of between about 0.008 to about 5 mg, e.g. of between about 0.03 mg to about 5 mg S1PR modulator per kg body weight per 1 to 2 days. Examples of suitable amounts within this range are 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 3, 3.5, 4, 4.5, and 5 mg ozanimod per kg body weight per 1 to 2 days.

As an not inventive example it is disclosed that fingolimod (FTY720) may be administered, for example, in an amount of between about 0.008 mg (corresponding to the 0.5 mg daily dose of multiple sclerosis patients) to about 3 mg fingolimod per kg body weight per 1 to 2 days, e.g. of between about 0.2 mg to about 3 mg fingolimod per kg body weight per 1 to 2 days or of between about 0.2 mg to about 1 mg fingolimod per kg body weight per 1 to 2 days. Examples of suitable amounts within this range are 0.008, 0.009, 0.01, 0.02, 0.03, 0.04, 0,05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, and 3 mg fingolimod per kg body weight per 1 to 2 days.

According to the invention ozanimod (RPC1063) may be administered in an amount of between about 0.008 mg (corresponding to the 0.5 mg daily dose of multiple sclerosis patients) to about 3 mg ozanimod per kg body weight per 1 to 2 days, e.g. of between about 0.2 mg to about 3 mg ozanimod per kg body weight per 1 to 2 days or of between about 0.2 mg to about 1 mg ozanimod per kg body weight per 1 to 2 days. Examples of suitable amounts within this range are 0.008, 0.009, 0.01, 0.02, 0.03, 0.04, 0,05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, and 3 mg ozanimod per kg body weight per 1 to 2 days.

Further disclosed is that siponimod (BAF312) may be administered, for example, in an amount of between about 0.008 mg to about 3 mg siponimod per kg body weight per 1 to 2 days, e.g. of between about 0.2 mg to about 3 mg siponimod per kg body weight per 1 to 2 days or of between about 0.2 mg to about 1 mg siponimod per kg body weight per 1 to 2 days. Examples of suitable amounts within this range are 0.008, 0.009, 0.01, 0.02, 0.03, 0.04, 0,05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, and 3 mg siponimod per kg body weight per 1 to 2 days.

Further disclosed is that ponesimod (ACT128800) may be administered, for example, in an amount of between about 0.008 mg to about 3 mg ponesimod per kg body weight per 1 to 2 days, e.g. of between about 0.2 mg to about 3 mg ponesimod per kg body weight per 1 to 2 days or of between about 0.2 mg to about 1 mg ponesimod per kg body weight per 1 to 2 days. Examples of suitable amounts within this range are 0.008, 0.009, 0.01, 0.02, 0.03, 0.04, 0,05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, and 3 mg ponesimod per kg body weight per 1 to 2 days.

The skilled person appreciates that depending on the individual patient situation, other dosages and/or administration periods may be suitable or required.

Accordingly, in an even more preferred embodiment, the sphingosine-1-phosphate receptor (S1PR) modulator for use in treating a patient suffering from Alzheimer's dementia in a late stage of Alzheimer's disease is ozanimod (RPC1063), wherein the Alzheimer's dementia is sporadic Alzheimer's dementia, wherein the patient is about 60 years or older, and wherein ozanimod is to be administered in an amount of between about 0.008 to about 5 mg, e.g. of between about 0.03 mg to about 5 mg ozanimod per kg body weight per 1 to 2 days.

In another even more preferred embodiment, the sphingosine-1-phosphate receptor (S1PR) modulator for use in treating a patient suffering from Alzheimer's dementia in a late stage of Alzheimer's disease is ozanimod (RPC1063), wherein the Alzheimer's dementia is familial Alzheimer's dementia, wherein the patient is about 40 years or older, and wherein ozanimod is to be administered in an amount of between about 0.008 to about 5 mg, e.g. of between about 0.03 mg to about 5 mg ozanimod per kg body weight per 1 to 2 days.

For example, ozanimod (RPC1063) is available as capsules with different strengths and should be taken once daily. To reduce the risk of side effects, the dose may be increased slowly at the beginning of treatment or after interruption of treatment. The starting dose may be one 0.23 mg capsule daily during the first 4 days, one 0.46 mg capsule daily during the following 3 days (on days 5, 6, and 7), and one 0.92 mg capsule daily starting on day 8.

According to the invention, the administration of the S1PR modulator ozanimod may take place, for example, over a period of about 1 month to 24 months, preferably over a period of about 1 month to 12 months, e.g., about 1 month to 6 months or about 1 month to 2 months. However, the skilled person appreciates that depending on the individual patient situation, other administration periods may be suitable or required. For example, the application could also be recurring rhythmic such as (daily, or every second day or once a week) application for 2 months, then suspend for 6 months, then again application for 2 months (in a different rhythm, e.g. once a week, every second day, daily, or with a different FTY720 concentration)), or any other related scheme consisting of recurring treatment periods with interleaved pausing of treatment.

In one embodiment of the invention, the patient to be administered with the S1PR modulator ozanimod may be a patient who has received/is receiving behavioral therapy, physical exercise training, reality orientation training, physiotherapy, occupational therapy, or cognitive training (memory training), or a combination thereof.

In another embodiment of the invention, the S1PR modulator ozanimod may be used in combination with a drug selected from the group consisting of aducanumab, lecanemab, gantenerumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

Aducanumab is an amyloid beta-directed monoclonal antibody approved in the USA in June 2021 under the name Aduhelm (Biogen) through an accelerated procedure for the treatment of Alzheimer's disease. Lecanemab is another amyloid beta-directed monoclonal antibody, which was very recently approved by the US Food and Drug Administration (FDA) for the treatment of Alzheimer's disease. Gantenerumab is also an amyloid beta-directed monoclonal antibody, which is under investigation.

Riluzole is approved in the USA for the treatment of amyotrophic lateral sclerosis (ALS) and has the formula:

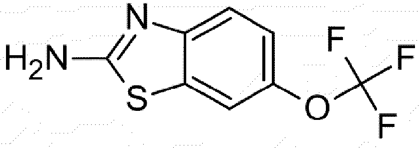

Donepezil is approved in the USA in 2006 for the treatment of mild, moderate, and severe dementia in Alzheimer's disease and has the formula:

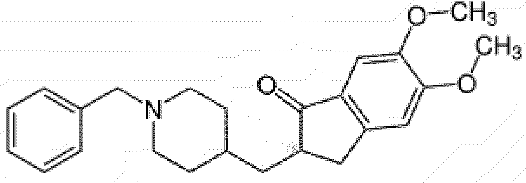

Fluoxetine, sold under the brand names Prozac and Sarafem among others, is an antidepressant. It is on the World Health Organization's List of Essential Medicines and has the formula:

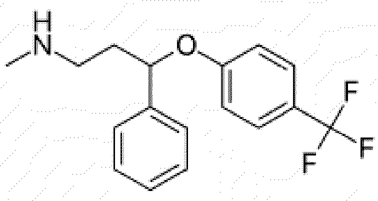

Citalopram, sold under the brand name Celexa among others, is an antidepressant. It is on the World Health Organization's List of Essential Medicines and has the formula:

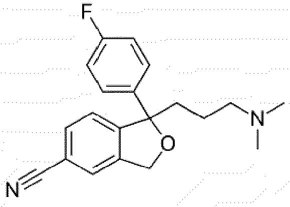

Methylene blue (methylthionium chloride) is a salt of a thiazine dye. It is a tau protein aggregation inhibitor, stabilizes the physiologically normal function of mitochondria and avoids the formation of toxic oxygen radicals. Its formula is:

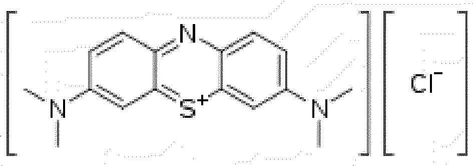

Curcumin is a bright yellow chemical produced by plants of the *Curcuma longa* species. It is a tau protein aggregation inhibitor and has the formula (keto tautomer):

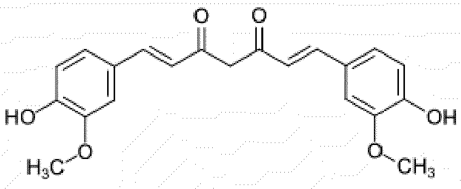

Lithium chloride is a tau protein aggregation inhibitor.

Roscovitine (seliciclib, CYC202) is *inter alia* under investigation for the treatment of chronic inflammation disorders. It is a tau protein aggregation inhibitor and has the formula:

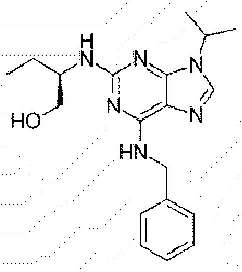

Memantine was approved for medical use in the USA in 2003 and is used to slow the progression of moderate to severe Alzheimer's disease. It is a tau protein aggregation inhibitor and has the formula:

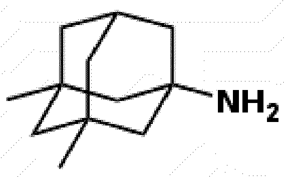

Sodium selenate is a tau protein aggregation inhibitor and has the formula Na₂SeO₄.

Pyridoxamine is one form of vitamin B6. It stabilizes the physiologically normal function of mitochondria and avoids the formation of toxic oxygen radicals. Its formula is:

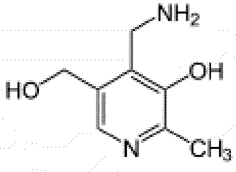

5-aminoimidazole-4-carboxamide-1-β-D-ribofuranoside (Acadesine, AICAR) is used for the treatment of acute lymphoblastic leukemia and has the formula:

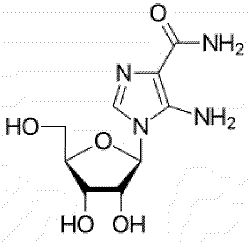

The skilled person appreciates that certain of the above compounds may have different tautomeric forms and/or different optical isomers as a result of one or more asymmetric or chiral carbon atoms. Further, some compounds may display polymorphism. It is also clear that pharmaceutically acceptable salts, esters, solvates, or hydrates of the above compounds as well as prodrugs that may convert *in vivo* into the above compounds may be used in the invention.

Examples for pharmacologically acceptable salts of the above compounds are salts of sufficiently basic compounds of the above formulas with physiologically acceptable mineral acids like hydrochloric, sulfuric or phosphoric acid; or salts of organic acids like acetic, lactic, citric, succinic, fumaric, maleic or salicylic acid. Further, sufficiently acid compounds of the above formulas may form alkali or earth alkaline metal salts, for example, sodium, potassium, lithium, calcium or magnesium salts; ammonium salts; or salts of an organic base, for example, methylamine, dimethylamine, trimethylamine, triethylamine, ethylenediamine, ethanolamine, choline hydroxide, piperidine, morpholine, tris-(2-hydroxyethyl)amine, lysine or arginine salts.

Pharmacologically acceptable esters of the above compounds are esters of the above compounds that can be hydrolyzed by esterase enzymes in the human body into compounds with the above formulas.

Pharmacologically acceptable solvates of the above compounds are complex compounds (adducts) formed by the interaction of the molecules of a solvent with molecules of the above compounds. Hydrates are solvates where the solvent is water.

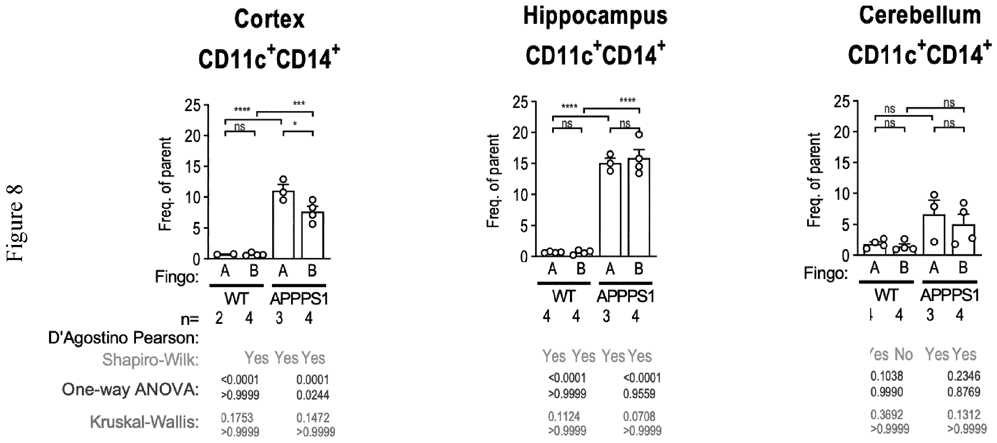

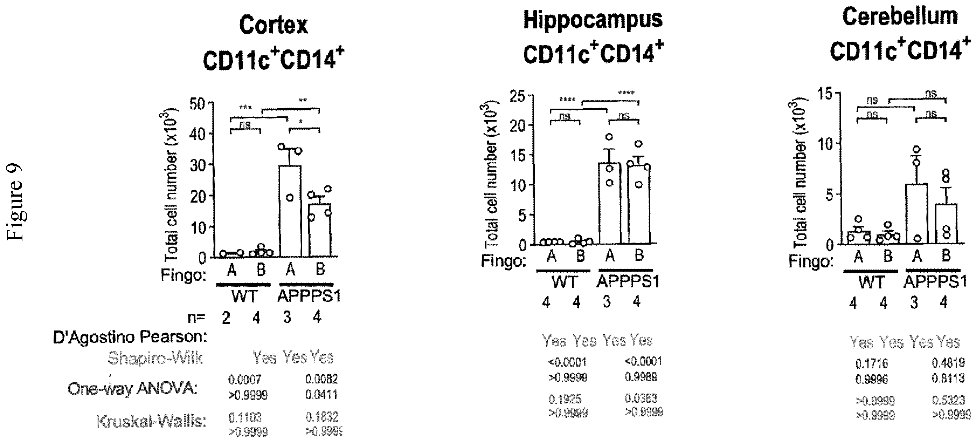

The present inventors found that the administration of fingolimod reduces the increased frequency and increased amount of CD11c⁺/CD14⁺ age/AD-associated microglia in the cortex of 15-month-old male AD mice (see Figures 8 and 9). Thus, it is reasonable to assume that fingolimod targets CD11c⁺/CD14⁺ age/AD-associated microglia in the cortex. Accordingly, it is disclosed but not part of the invention that the sphingosine-1-phosphate receptor (S1PR) modulator such as fingolimod as described herein is for use in treating a patient suffering from Alzheimer's dementia, wherein fingolimod targets CD11c⁺/CD14⁺ age/AD-associated microglia in the cortex of the patient.

The first aspect of the present invention can alternatively be formulated as follows: Use of a sphingosine-1-phosphate receptor (S1PR) modulator, namely ozanimod, for the manufacture of a medicament for the treatment of a patient suffering from Alzheimer's dementia in a late stage of Alzheimer's disease. Moreover, the first aspect of the present invention can alternatively be formulated as follows: A S1PR modulator for use in treating a patient suffering from Alzheimer's dementia in a late stage of Alzheimer's disease, wherein said S1PR modulator is ozanimod.

In a second aspect, the invention relates to a combination for use in treating a patient suffering from Alzheimer's dementia in a late stage of Alzheimer's disease, wherein the combination comprises
(i) an S1PR modulator, and
(ii) another therapeutic substance, wherein
   the S1PR modulator is ozanimod (RPC1063). The following S1PR modulators are also known in the art: fingolimod (FTY720), siponimod (BAF312), ceralifimod (ONO-4641), GSK2018682, ponesimod (ACT128800), and amiselimod (MT-1303).

The other therapeutic substance is also suitable to treat Alzheimer's dementia in a patient.

In a more preferred embodiment, the combination for use in treating a patient suffering from Alzheimer's dementia in a late stage of Alzheimer's disease comprises
(i) an S1PR modulator, and
(ii) another therapeutic substance suitable to treat Alzheimer's dementia,
wherein the sphingosine-1-phosphate receptor (S1PR) modulator is ozanimod (RPC1063), wherein the the Alzheimer's dementia is sporadic Alzheimer's dementia and wherein the patient is about 60 years or older.

In another more preferred embodiment, the combination for use in treating a patient suffering from Alzheimer's dementia in a late stage of Alzheimer's disease comprises
(i) an S1PR modulator, and
(ii) another therapeutic substance suitable to treat Alzheimer's dementia,
wherein the sphingosine-1-phosphate receptor (S1PR) modulator is ozanimod (RPC1063), wherein the Alzheimer's dementia is familial Alzheimer's dementia and wherein the patient is about 40 years or older.

The other therapeutic substance may be selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

Preferred combinations of the S1PR modulator and the other therapeutic substance are:
- Ozanimod and Fluoxetine
- Ozanimod and Citalopram
- Ozanimod and AICAR
- Ozanimod and sodium selenite
- Ozanimod and LiCl
- Ozanimod and Lecanemab
- Ozanimod and Gantenerumab
- Ozanimod and Aducanumab

The components of the combination, i.e. the S1PR modulator and the other therapeutic substance, may be administered together or independent from each other (e.g. one after the other).

In an even more preferred embodiment, the combination for use in treating a patient suffering from Alzheimer's dementia in a late stage of Alzheimer's disease comprises
(i) an S1PR modulator, and
(ii) another therapeutic substance suitable to treat Alzheimer's dementia,
wherein the sphingosine-1-phosphate receptor (S1PR) modulator is ozanimod (RPC1063), wherein the other therapeutic substance suitable to treat Alzheimer's dementia is selected from the group consisting of fluoxetine, citalopram, AICAR, sodium selenite, LiCl, Lecanemab, and Gantenerumab, wherein the Alzheimer's dementia is sporadic Alzheimer's dementia, and wherein the patient is about 60 years or older.
In the above embodiment, ozanimod is particularly to be administered in an amount of between about 0.008 to about 5 mg, e.g. of between about 0.03 mg to about 5 mg ozanimod per kg body weight per 1 to 2 days.

In another even more preferred embodiment, the combination for use in treating a patient suffering from Alzheimer's dementia in a late stage of Alzheimer's disease comprises
(i) an S1PR modulator, and
(ii) another therapeutic substance suitable to treat Alzheimer's dementia,
wherein the sphingosine-1-phosphate receptor (S1PR) modulator is ozanimod (RPC1063), wherein the other therapeutic substance suitable to treat Alzheimer's dementia is selected from the group consisting of fluoxetine, citalopram, AICAR, sodium selenite, LiCl, Lecanemab, and Gantenerumab, wherein the Alzheimer's dementia is familial Alzheimer's dementia, and wherein the patient is about 40 years or older.
In the above embodiment, ozanimod is particularly to be administered in an amount of between about 0.008 to about 5 mg, e.g. of between about 0.03 mg to about 5 mg ozanimod per kg body weight per 1 to 2 days.

As to other preferred embodiments, it is referred to the first aspect of the present invention.

The second aspect of the present invention can alternatively be formulated as follows: Use of a combination of
(i) an S1PR modulator, and
(ii) another therapeutic substance,
wherein said S1PR modulator is ozanimod (RPC1063), for the manufacture of a medicament for the treatment of a patient suffering from Alzheimer's dementia in a late stage of Alzheimer's disease.

In a third aspect, the present invention relates to a pharmaceutical composition comprising the sphingosine-1-phosphate receptor (S1PR) modulator ozanimod as defined in the first aspect or the combination as defined in the second aspect for use in treating a patient suffering from Alzheimer's dementia in a late stage of Alzheimer's disease.

Preferably, the pharmaceutical composition comprises one or more pharmaceutically acceptable excipients, diluents, and/or carriers.

As to preferred embodiments, it is referred to the first or second aspect of the present invention.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention, which are obvious to those skilled in the art in the relevant fields are intended to be covered by the present invention.

### BRIEF DESCRIPTION OF THE FIGURES

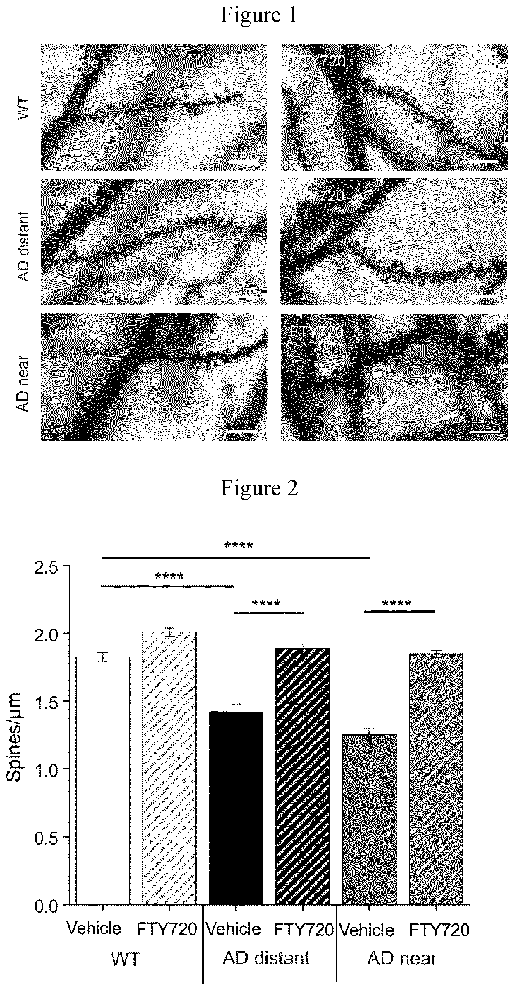

The following Figures are merely illustrative of the present invention and should not be construed to limit the scope of the invention as indicated by the appended claims in any way. Furthermore, figures 1 to 6 as well as figures 8 and 9 disclose experiments performed by the inventors with fingolimod as illustrative examples, that do not fall within the scope of the claims.
**FIGURE** 1 shows microscope photographs of Fingolimod (FTY720) restored spine deficits in CA1 pyramidal neurons in male 15-month-old AD model mice (Radde et al., 2006).
**FIGURE** 2 shows the quantitative results of a corresponding dendritic spine density analysis.
**FIGURE 3** shows that Fingolimod (FTY720) restored deficit in contextual fear learning in 15-month-old male AD mice (Radde et al., 2006). Dose FTY720: 3.0 mg/kg/2nd day for 1 month.
**FIGURE 4** shows that Fingolimod (FTY720) restored spine deficits in CA3 pyramidal neurons and in dentate gyrus granule cells in 15-month-old male AD mice (Radde et al., 2006). Dose FTY720: 1.0 mg/kg/2nd day for 1 month.
**FIGURE 5** shows that Fingolimod (FTY720) restored spine deficits in CA3 pyramidal neurons and in dentate gyrus granule cells in 15-month-old male AD mice (Radde et al., 2006). Dose FTY720: 3.0 mg/kg/2nd day for 1 month.
**FIGURE 6** shows that a low dose of Fingolimod (FTY720) restored spine deficits in CA1 pyramidal neurons in 6-7-month-old male AD mice (Radde et al., 2006). Low dose FTY720: 0.2 mg/kg/2nd day for 1 month.
**FIGURE 7** shows that chronic (4 weeks) Ozanimod treatment according to the invention restored spine deficits in CA1 pyramidal neurons in aged (16-month-old) male AD mice (Radde et al., 2006). Dose Ozanimod: 1 mg/kg via drinking water for 1 month.
**FIGURE 8** shows that Fingolimod (FTY720) reduces the increased frequency of CD11c⁺/CD14⁺ age/AD-associated microglia in the cortex of 15-month-old male AD mice.
   WT: wild-type littermates
   APP/PS1: AD mice (Radde et al, 2006)
   Substance A: Control
   Substance B: FTY720 (1.0 mg/kg/day) for 1 month.
**FIGURE 9** shows that Fingolimod (FTY720) reduces the increased number of CD11c⁺/CD14⁺ age/AD-associated microglia in the cortex of 15-month-old male AD mice.
   WT: wild-type littermates
   APP/PS1: AD mice (Radde et al, 2006)
   Substance A: Control
   Substance B: FTY720 (1.0 mg/kg/day) for 1 month.

### EXAMPLES

The examples given below are for illustrative purposes only and do not limit the invention described above in any way.

The following examples with fingolimod are only illustrative examples of experiments made by the inventors and do not fall within the scope of the claims.

The therapeutic effect of fingolimod (FTY720) as an example for an S1PR modulator for use in the treatment of Alzheimer's disease was tested in an established double transgenic amyloid precursor protein/presenilin-1(APP/PS1) Alzheimer's disease (AD) mouse model that starts to develop amyloid-β (Aβ) pathology in the neocortex and hippocampus at two and four months of age, respectively. This AD mouse model is particularly close to human AD pathology. Impaired LTP (long-term potentiation) at Schaffer collateral CA1 synapses in acute slices *ex vivo* is observed at five months in these mice and becomes detectable with *in vivo* recordings beyond six months of age. Moreover, hippocampus-dependent learning deficits in this APP/PS1 mouse model become apparent five to eight months after birth.

To test for a possible amelioration of the AD phenotype at a late symptomatic stage in the above APP/PS1 mouse model, fingolimod (FTY720) treatment was started at fifteen months of age, when massive loss of synapses and memory has already occurred.

Male APP/PS1 mice and littermate controls (wild-type healthy mice) were treated with intraperitoneal (i.p.) injections of fingolimod (FTY720; dissolved in 3% DMSO, 1.0 mg/kg or 3.0 mg/kg body weight) every second day for 1 to 2 months.

The analyses of the animals comprises dendritic spine density analysis, LTP recordings, Morris water maze spatial learning and memory testing, contextual fear condition training and memory testing, immunohistological stainings of microgliosis and astrogliosis, biochemical brain-derived neurotrophic factor (BDNF) quantification and downstream TrkB receptor (a high affinity BDNF receptor) signaling, and statistical analysis.

Spine density was determined with Golgi-Cox staining of hippocampal cornu ammonis-1 (CA1) pyramidal neurons. In the APP/PS1 mice, amyloid-β (Aβ) plaques were visualized with the blue fluorescent dye Methoxy X-04.

FIGURE 1 shows secondary apical dendrites of CA1 pyramidal neurons from 15 months old mice in vehicle and fingolimod treated wild type (WT) animals (WT, upper panel), in APP/PS1 animals >50 µm away from the closest plaque (AD distant, middle panel), or in APP/PS1 mice <50 µm away from the nearest plaque (AD near, lower panel). Scale bar: 5 µm in all images.

FIGURE 2 shows the quantification of spine densities in secondary apical CA1 dendrites shown for the six different groups in Figure 1. Each bar represents the mean value of 10 dendrites from 10 different CA1 pyramidal neurons per animal (three animals per group). Horizontal bars with asterisks indicate statistical significance between selected groups. Significance of differences was tested with two-way ANOVA followed by Tukey's post hoc test (n = 30 dendrites from three animals per group). Significance level was set to 0.05 (p < 0.05). Different levels of significance are indicated by asterisks, with * * = p < 0.01, * * * = p < 0.001.

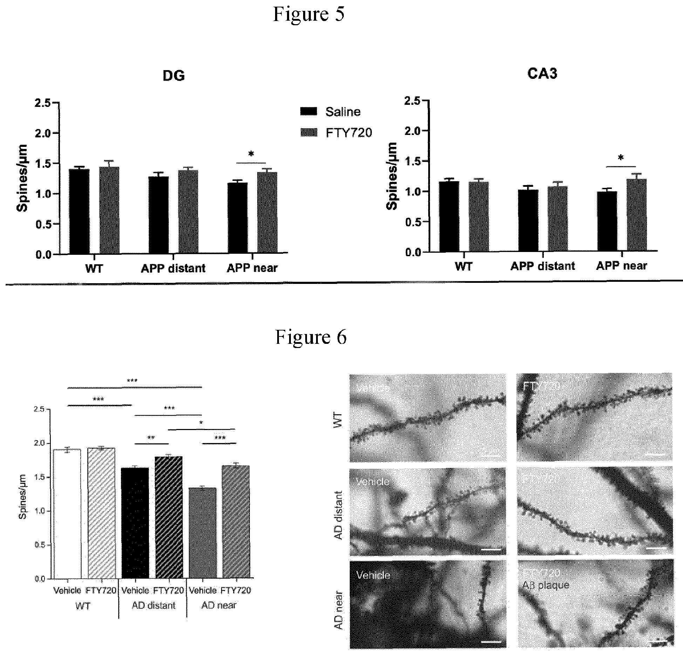

An identical effect of spine rescue in APP/PS1 mice was also observed with much lower doses of fingolimod (FTY720), e.g., 0.2 mg/kg body weight, were applied with the same treatment regime (see Figure 6).

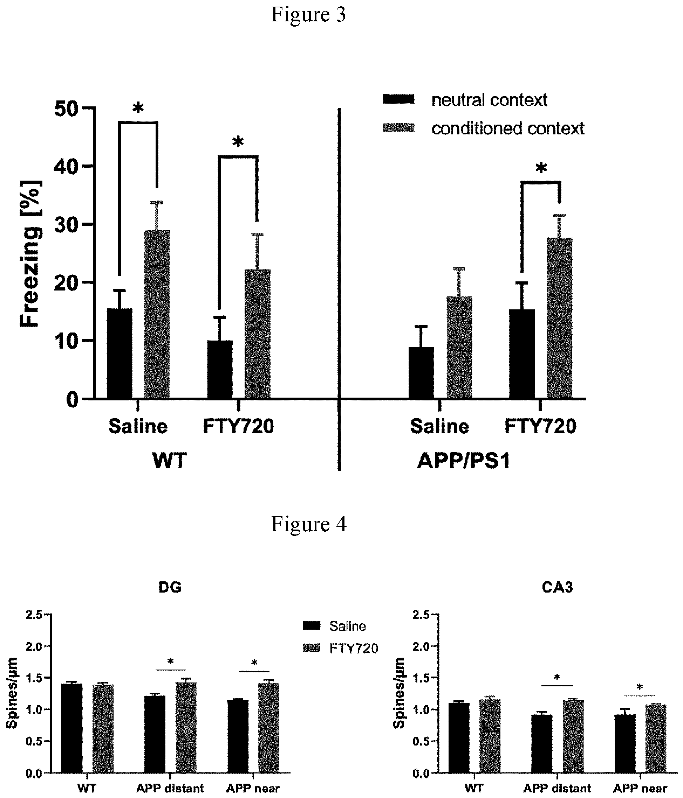

The Figures show the complete rescue of spine deficits in dendrites distant to plaques in fingolimod-treated APP/PS1 mice, and the significant amelioration of spine deficits in dendrites close to plaques (see Figures 1 and 2; 6-7 months old AD mice). In dentate gyrus granule cells and CA3 pyramidal neurons of 15-16-months old AD mice, 1 month FTY treatment completely rescued spine decrease near and distant to Aβ plaques (see Figure 4). The number of synaptic spines can be restored at both ages (6-7 months and 15-16 months) to the level of same-aged healthy mice (wild-type littermates) showing that short-term fingolimod (FTY720) treatment leads to reversion of Alzheimer's disease symptoms when massive loss of synapses and memory has already occurred (see Figures 1, 2, 4, 5, 6). Fingolimod reduces the increased frequency and increased number of CD11c⁺/CD14⁺ age/AD-associated microglia in the cortex of 15-month-old male AD mice (see Figures 8 and 9).

FIGURE 3 shows that Fingolimod (FTY720) restored the deficit in contextual fear learning in 15-month-old male AD mice. Dose FTY720: 3.0 mg/kg/2nd day for 1 month.

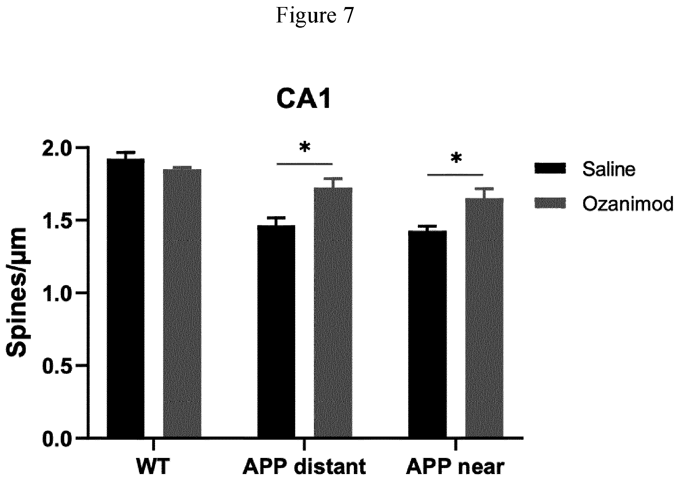

FIGURE 7 shows that according to the invention the S1PR modulator Ozanimod has a very similar capacity to restore spine deficits in CA1 pyramidal neurons in aged (16-month-old) male AD mice. Dose Ozanimod: 1 mg/kg via drinking water for 1 month, starting at 15 months.

FIGURE 4 shows that Fingolimod (FTY720) restored spine deficits in CA3 and dentate gyrus pyramidal neurons in 15-month-old male AD mice. Dose FTY720: 1.0 mg/kg/2nd day for 1 month.

FIGURE 5 shows that Fingolimod (FTY720) restored spine deficits in CA3 and dentate gyrus pyramidal neurons in 15-month-old male AD mice. Dose FTY720: 3.0 mg/kg/2nd day for 1 month.

FIGURE 6 shows that a low dose of Fingolimod (FTY720) restored spine deficits in CA1 pyramidal neurons in 6-7-month-old male AD mice. Low dose FTY720: 0.2 mg/kg/2nd day for 1 month.

FIGURE 8 shows that Fingolimod (FTY720) reduces the increased frequency of CD11c⁺/CD14⁺ age/AD-associated microglia in the cortex of 15-month-old male AD mice. WT: wild-type littermates, APP/PS1: AD mice (Radde et al, 2007)
Substance A: Control, Substance B: FTY720 (1.0 mg/kg/2nd day) for 1 month.

FIGURE 9 shows that Fingolimod (FTY720) reduces the increased number of CD11c⁺/CD14⁺ age/AD-associated microglia in the cortex of 15-month-old male AD mice. WT: wild-type littermates, APP/PS1: AD mice (Radde et al, 2007), Substance A: Control, Substance B: FTY720 (1.0 mg/kg/2nd day) for 1 month.

While certain representative embodiments and details have been shown to illustrate the present invention, it will be apparent to those skilled in this art that various changes and modifications can be made and that, within the scope of the appended claims, the invention may be practiced otherwise than as specifically described and claimed.

### REFERENCES

Radde R, Bolmont T, Kaeser SA, Coomaraswamy J, Lindau D, Stoltze L, Calhoun ME, Jäggi F, Wolburg H, Gengler S, Haass C, Ghetti B, Czech C, Hölscher C, Mathews PM, Jucker M. Abeta42-driven cerebral amyloidosis in transgenic mice reveals early and robust pathology. EMBO Rep. 2006 Sep;7(9):940-6. doi: 10.1038/sj.embor.7400784. Epub 2006 Aug 11. PMID: 16906128; PMCID: PMC1559665.

## Claims

1. A sphingosine-1-phosphate receptor (S1PR) modulator for use in treating a patient suffering from Alzheimer's dementia in a late stage of Alzheimer's disease, wherein said S1PR modulator is ozanimod (RPC1063).

2. The S1PR modulator for use according to claim 1, wherein said patient suffering from Alzheimer's dementia is about 40 years old or older.

3. The S1PR modulator for use according to claim 1 or 2, wherein the Alzheimer's dementia is sporadic Alzheimer's dementia or familial Alzheimer's dementia.

4. The S1PR modulator for use according to any one of claims 1 to 3, wherein said treatment is effective to reverse Alzheimer's dementia.

5. The S1PR modulator for use according to any one of claims 1 to4, wherein said S1PR modulator is to be administered intraperitoneally or orally.

6. The S1PR modulator for use according to any one of claims 1 to 5, wherein ozanimod is to be administered in an amount of between 0.008 mg to 5 mg S1PR modulator per kg body weight per 1 to 2 days.

7. The S1PR modulator for use according to claim 6, wherein ozanimod is to be administered in an amount of between 0.008 mg to 3 mg ozanimod per kg body weight per 2 days.

8. The S1PR modulator for use according to claims 6 or 7, wherein said administration takes place over a period of about 1 month to 24 months, preferably over a period of about 1 month to 12 months, e.g. about 1 month to 6 months, or about 1 month to 2 months.

9. The S1PR modulator for use according to any one of claims 1 to 8, wherein the patient to be administered is a patient who has received/is receiving behavioral therapy, reality orientation training, physiotherapy, occupational therapy, or cognitive training (memory training), or a combination thereof.

10. The S1PR modulator for use according to any one of claims 1 to 9, wherein the S1PR modulator is used in combination with a drug selected from the group consisting of aducanumab, lecanemab, gantenerumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribo-furanoside (AICAR).

11. A combination for use in treating a patient suffering from Alzheimer's dementia in a late stage of Alzheimer's disease, wherein the combination comprises
(i) an S1PR modulator, and
(ii) another therapeutic substance,
wherein said S1PR modulator is ozanimod (RPC1063).

12. The combination for use according to claim 11, wherein the other therapeutic substance is selected from the group consisting of aducanumab, lecanemab, gantenerumab, riluzole, donepezil, fluoxetine, citalopram, methylene blue, curcumin, lithium chloride, roscovitine, memantine, sodium selenate, pyridoxamine (vitamin B6), and 5-aminoimidaz-ole-4-carboxamide-1-β-D-ribofuranoside (AICAR).

13. A pharmaceutical composition comprising the sphingosine-1-phosphate receptor (S1PR) modulator as defined in claims 1 to 10 or the combination as defined in claims 11 to 12 for use in treating a patient suffering from Alzheimer's dementia in a late stage of Alzheimer's disease.

## Patentansprüche

1. Sphingosin-1-phosphat-Rezeptor- (S1PR) -Modulator zur Verwendung bei der Behandlung eines Patienten, der an Alzheimer-Demenz in einem späten Stadium der Alzheimer-Erkrankung leidet, wobei der genannte S1PR-Modulator Ozanimod (RPC1063) ist.

2. S1PR-Modulator zur Verwendung gemäß Anspruch 1, wobei der genannte Patient, der an Alzheimer-Demenz leidet, ungefähr 40 Jahre alt oder älter ist.

3. S1PR-Modulator zur Verwendung gemäß Anspruch 1 oder 2, wobei die Alzheimer-Demenz sporadische Alzheimer-Demenz oder familiäre Alzheimer-Demenz ist.

4. S1PR-Modulator zur Verwendung gemäß einem der Ansprüche 1 bis 3, wobei die genannte Behandlung wirksam ist, die Alzheimer-Demenz zurückzubilden.

5. S1PR-Modulator zur Verwendung gemäß einem der Ansprüche 1 bis 4, wobei der genannte S1PR-Modulator intraperitoneal oder oral zu verabreichen ist.

6. S1PR-Modulator zur Verwendung gemäß einem der Ansprüche 1 bis 5, wobei Ozanimod in einer Menge von zwischen 0,008 mg bis 5 mg S1PR-Modulator pro kg Körpergewicht pro ein bis 2 Tage zu verabreichen ist.

7. S1PR-Modulator zur Verwendung gemäß Anspruch 6, worin Ozanimod in einer Menge von zwischen 0,008 mg bis 3 mg Ozanimod pro kg Körpergewicht pro 2 Tage zu verabreichen ist.

8. S1PR-Modulator zur Verwendung gemäß den Ansprüchen 6 oder 7, wobei die genannte Verabreichung über einen Zeitraum von ungefähr einem Monat bis 24 Monate, bevorzugt über einen Zeitraum von ungefähr einem Monat bis 12 Monate, z. B. ungefähr einem Monat bis 6 Monate, oder ungefähr einem Monat bis 2 Monate.

9. S1PR-Modulator zur Verwendung gemäß einem der Ansprüche 1 bis 8, wobei der Patient, dem zu verabreichen ist, ein Patient ist, der Verhaltenstherapie, Realitätsorientierungstraining, Physiotherapie, Ergotherapie oder kognitives Training (Gedächtnistraining) oder eine Kombination davon erhielt/erhält.

10. S1PR-Modulator zur Verwendung gemäß einem der Ansprüche 1 bis 9, wobei der S1PR-Modulator in Kombination mit einem Arzneimittel verwendet wird, ausgewählt aus der Gruppe, bestehend aus Aducanumab, Lecanemab, Gantenerumab, Riluzol, Donepezil, Fluoxetin, Citalopram, Methylenblau, Curcumin, Lithiumchlorid, Roscovitin, Memantin, Natriumselenat, Pyridoxamin (Vitamin B6) und 5-Aminoimidazol-4-carboxamid-1-β-D-ribofuranosid (AICAR).

11. Kombination zur Verwendung bei der Behandlung eines Patienten, der an Alzheimer-Demenz in einem späten Stadium der Alzheimer-Krankheit leidet, wobei die Kombination
(i) einen S1PR-Modulator und
(ii) eine andere therapeutische Substanz
umfasst, wobei der genannte S1PR-Modulator Ozanimod (RPC1063) ist.

12. Kombination zur Verwendung gemäß Anspruch 11, wobei die andere therapeutische Substanz ausgewählt ist aus der Gruppe bestehend aus Aducanumab, Lecanemab, Gantenerumab, Riluzol, Donepezil, Fluoxetin, Citalopram, Methylenblau, Curcumin, Lithiumchlorid, Roscovitin, Memantin, Natriumselenat, Pyridoxamin (Vitamin B6) und 5-Aminoimidazol-4-carboxamid-1-β-D-ribofuranosid (AICAR).

13. Pharmazeutische Zusammensetzung umfassend den Sphingosin-1-phosphat-Rezeptor-(S1PR) -Modulator wie in den Ansprüchen 1 bis 10 definiert, oder die Kombination wie in den Ansprüchen 11 bis 12 definiert, zur Verwendung bei der Behandlung eines Patienten, der an Alzheimer-Demenz in einem späten Stadium der Alzheimer-Erkrankung leidet.

## Revendications

1. Un modulateur du récepteur de la sphingosine-1-phosphate (S1PR) destiné à être utilisé dans le traitement d'un patient souffrant de démence d'Alzheimer à un stade avancé de la maladie d'Alzheimer, ledit modulateur du S1PR étant l'ozanimod (RPC1063).

2. Le modulateur du S1PR destiné à être utilisé selon la revendication 1, ledit patient souffrant de démence d'Alzheimer étant âgé d'environ 40 ans ou plus.

3. Le modulateur du S1PR destiné à être utilisé selon la revendication 1 ou la revendication 2, la démence d'Alzheimer étant une démence d'Alzheimer sporadique ou une démence d'Alzheimer familiale.

4. Le modulateur du S1PR destiné à être utilisé selon l'une quelconque des revendications 1 à 3, ledit traitement étant efficace pour inverser la démence d'Alzheimer.

5. Le modulateur du S1PR destiné à être utilisé selon l'une quelconque des revendications 1 à 4, ledit modulateur du S1PR devant être administré par voie intrapéritonéale ou par voie orale.

6. Le modulateur du S1PR destiné à être utilisé selon l'une quelconque des revendications 1 à 5, l'ozanimod devant être administré à raison de 0,008 mg à 5 mg de modulateur du S1PR par kg de poids corporel tous les 1 à 2 jours.

7. Le modulateur du S1PR à utiliser selon la revendication 6, l'ozanimod devant être administré à raison de 0,008 mg à 3 mg d'ozanimod par kg de poids corporel tous les 2 jours.

8. Le modulateur du S1PR destiné à être utilisé selon l'une des revendications 6 ou 7, ladite administration s'étendant sur une période d'environ 1 mois à 24 mois, de préférence sur une période d'environ 1 mois à 12 mois, par exemple d'environ 1 mois à 6 mois, ou d'environ 1 mois à 2 mois.

9. Le modulateur du S1PR destiné à être utilisé selon l'une quelconque des revendications 1 à 8, le patient auquel il est administré étant un patient qui a reçu ou qui reçoit une thérapie comportementale, un entraînement à l'orientation dans la réalité, de la kinésithérapie, de l'ergothérapie ou un entraînement cognitif (entraînement de la mémoire), ou une combinaison de ceux-ci.

10. Le modulateur du S1PR destiné à être utilisé selon l'une quelconque des revendications 1 à 9, le modulateur du S1PR étant utilisé en association avec un médicament choisi dans le groupe constitué par l'aducanumab, le lécanemab, le gantenerumab, le riluzole, le donépézil, la fluoxétine, le citalopram, le bleu de méthylène, la curcumine, le chlorure de lithium, la roscovitine, la mémantine, le sélénate de sodium, la pyridoxamine (vitamine B6) et le 5-aminoimidazole-4-carboxamide-1-β-D-ribofuranoside (AICAR).

11. Association destinée à être utilisée dans le traitement d'un patient souffrant de démence d'Alzheimer à un stade avancé de la maladie d'Alzheimer, l'association comprenant
(i) un modulateur du S1PR, et
(ii) une autre substance thérapeutique,
ledit modulateur du S1PR étant l'ozanimod (RPC1063).

12. L'association destinée à être utilisée selon la revendication 11, dans laquelle l'autre substance thérapeutique est choisie dans le groupe constitué par l'aducanumab, le lécanemab, le gantenerumab, le riluzole, le donépézil, la fluoxétine, le citalopram, le bleu de méthylène, la curcumine, le chlorure de lithium, la roscovitine, la mémantine, le sélénate de sodium, la pyridoxamine (vitamine B6) et le 5-aminoimidazole-4-carboxamide-1-β-D-ribofuranoside (AICAR).

13. Composition pharmaceutique comprenant le modulateur du récepteur de la sphingosine-1-phosphate (S1PR) tel que défini dans les revendications 1 à 10 ou l'association telle que définie dans les revendications 11 à 12, destinée à être utilisée dans le traitement d'un patient souffrant de démence d'Alzheimer à un stade avancé de la maladie d'Alzheimer.
